# DEMANDE DE BREVET EUROPEEN

(11) **EP 0 738 736 A1**
(43) Date de publication de la demande: **23.10.1996**
(21) Numéro de dépôt: 96400799.1
(22) Date de dépôt: 15.04.1996
(51) Int. Cl.: C07K 16/28, A61K 39/395, G01N 33/577

(54) **Anticorps monoclonaux anti-gp130, et leurs utilisations**

(30) Priorité: 21.04.1995 FR 9504809
(71) Demandeur: DIACLONE S.A. à Directoire et Conseil de Surveillance, 25020 Besancon (FR)
(72) Inventeur: Wijdenes, John, 25720 Larnod (FR); Clement, Claude, 70100 Gray (FR)
(74) Mandataire: Vialle-Presles, Marie José

(57) **Abrégé**

L'Invention est relative à des anticorps monoclonaux anti-gp130, ainsi qu'à leurs utilisations pour l'obtention de médicaments, d'adjuvants de cultures cellulaires, et de réactifs de diagnostic.

## Description

L'Invention est relative à de nouveaux anticorps monoclonaux qui reconnaissent la glycoprotéine gp130, et qui peuvent induire la prolifération de lignées cellulaires possédant cet antigène, ainsi qu'à l'utilisation desdits anticorps monoclonaux pour le diagnostic et pour l'obtention de médicaments.

La gp130 est une glycoprotéine transmembranaire ubiquitaire, qui constitue un transducteur de signal commun à différentes cytokines, appartenant à la famille de l'IL-6 (interleukine-6). Outre l'IL-6, les cytokines concernées sont le CNTF (Ciliary Neurotropic Factor), l'IL-11 (interleukine-11), le LIF (Leukemia Inhibitory Factor), l'OM (Oncostatine M) [HIRANO et al. Stem Cells, 12, 262-277, (1994) ; ZHANG et al., J. Exp. Med., 177, 1337-1342 (1994)], la cardiotrophine I, [PENNICA et al., Proc. Natl. Acad. Sci. USA, 92, 1142-1146, (1995)], etc...

Certaines de ces cytokines (IL-11, LIF, OM) se fixent directement sur gp130.

D'autres, et en particulier l'IL-6, s'associent préalablement avec un récepteur spécifique présent sur les membranes cellulaires. Ce récepteur spécifique, dénommé gp80 ou IL-6R, a été décrit par TAGA et al. [J. Exp. Med., 166, 967-981, (1987)].

La transmission du signal implique d'abord la formation d'un complexe IL-6/IL-6R, qui s'associe ensuite à la gp130, ce qui induit l'homodimérisation, puis la phosphorylation de cette dernière [MURAKAMI et al., Science, 260, 1808-1810, (1993)] .

L'IL-6, et les cytokines de la même famille sont connues pour avoir un large spectre de fonctions biologiques ; elles interviennent en particulier dans la réponse immune, l'hématopoièse, le système nerveux, etc... En conséquence, de nombreux travaux ont pour but l'obtention de molécules pouvant moduler l'activité de ces cytokines.

De telles molécules ont en effet un rôle important à jouer pour étudier les mécanismes régissant la transmission de signaux inter- et intra-cellulaires, et pour le traitement des nombreuses maladies dans lesquelles interviennent lesdites cytokines.

C'est ainsi que l'équipe des Inventeurs a obtenu précédemment (Brevet Francais 2 694 767 au nom de INNOTHERAPIE LABORATOIRES S.A.) des anticorps monoclonaux reconnaissant le récepteur IL-6R, et capables d'inhiber l'interaction de l'IL-6 avec l'IL-6R, et la prolifération de lignées cellulaires IL-6 dépendantes.

TAGA et al. [Proc. Natl. Acad. Sci. USA, 89, 10998-11001 (1992)] décrivent des anticorps monoclonaux anti-gp130, bloquant la fixation du complexe IL-6/IL-6R sur la gp130, et la prolifération cellulaire (évaluée par la néosynthèse d'ADN), induite par l'activation de la gp130 qui en résulte.

Les Inventeurs ont maintenant isolé de nouvelles lignées cellulaires d'hybridomes produisant des anticorps monoclonaux anti-gp130, et ont constaté que, de manière tout à fait surprenante, certains de ces anticorps étaient capables de mimer l'action effectrice des différentes cytokines dont les signaux sont transmis par gp130.

La présente Invention a pour objet des anticorps monoclonaux dirigés contre le récepteur gp130, lesquels anticorps sont caractérisés en ce qu'ils sont choisis dans le groupe constitué par:
- l'anticorps monoclonal d'isotype IgG1 dénommé B-S12, produit par la lignée d'hybridome déposée le 12 avril 1995 auprès de la C.N.C.M. (COLLECTION NATIONALE DE CULTURES DE MICROORGANISMES, tenue par l'INSTITUT PASTEUR, 28, rue du Docteur Roux, 75724 PARIS CEDEX 15 - FRANCE), sous le numéro de Dépôt I-1561 ;
- l'anticorps monoclonal d'isotype IgG1 dénommé B-P8, produit par la lignée d'hybridome déposée le 12 avril 1995 auprès de la C.N.C.M., sous le numéro de Dépôt I-1560.

L'invention englobe également des variants de commutation de classe des anticorps ci-dessus, tels que par exemple des variants appartenant aux isotypes IgG3, IgG1, IgG2b, IgG2a et autres sous-classes d'immunoglobulines ; de tels variants peuvent être obtenus, par exemple, par le procédé décrit par COCO MARTIN et al. [J. Immunol.Methods. 145, p.1118, (1991)].

Les Inventeurs ont constaté que les anticorps monoclonaux conformes à l'Invention induisent, comme le complexe IL-6/IL-6R, la phosphorylation de gp130 ; or, on sait que cette phosphorylation nécessite la dimérisation préalable de gp130 [MURAKAMI et al., Science, (1993), publication précitée]. Il est probable que les anticorps monoclonaux conformes à l'Invention agissent, selon un mécanisme similaire à celui du complexe IL-6/IL-6R, en induisant l'homodimérisation, puis la phosphorylation de gp130.

Ce mécanisme d'action supposé expliquerait l'observation faite par les Inventeurs selon laquelle des fragments Fab de B-S12 reconnaissent gp130, mais n'ont pas d'effet biologique.

Les anticorps monoclonaux B-S12 et B-P8 reconnaissent des épitopes différents : l'épitope reconnu par B-S12 est situé dans la région dénommée boîte WSXWS [BAZAN, Proc. Natl. Acad. Sci..USA, 87, 6934-6938 (1989) ; GEARING et al., EMBO J., 8, 3667-3676 (1989)].

Du fait de leur mécanisme d'action similaire à celui du complexe IL-6/IL-6R, les anticorps monoclonaux conformes à l'Invention peuvent être utilisés, comme le complexe IL-6/IL-6R [YOSHIDA et al. Mechanisms of Development, 45, 163-171(1994)], pour la mise et le maintien en culture de cellules-souche portant gp130, telles que par exemple des cellules-souche hématopoiétiques, [BERARDI et al., Science, 267, 104-107, (1995)] des cellules-souche embryonnaires pluripotentes [NICHOLS et al., Experimental Cell Research, 215, 237-239 (1994)], etc...

Pour cette utilisation, les anticorps monoclonaux conformes à l'Invention peuvent être employés à des concentrations comprises entre 1 et 25 µg/ml, de préférence 10 µg/ml ; ils sont additionnés au milieu de culture au début de celle-ci.

Les anticorps monoclonaux conformes à l'Invention peuvent également être utilisés dans des pathologies pour lesquelles les cytokines dont les signaux sont transmis par l'intermédiaire de gp130, ont un effet bénéfique et peuvent apporter une amélioration.

Les anticorps monoclonaux conformes à l'Invention peuvent être utilisés seuls ou bien couplés à des toxines, ou à des substances radioactives ou à tout autre agent thérapeutique. Ils peuvent également être encapsulés dans des liposomes.

Des préparations pharmaceutiques comprenant des anticorps monoclonaux conformes à l'Invention peuvent se présenter sous forme liquide, ou sous forme lyophilisée. On peut utiliser pour stabiliser ces préparations pharmaceutiques, des protéines, des sucres, des sucres-alcools, des acides aminés ; pour les tamponner, des sels inorganiques, de préférence du phosphate de sodium dans un sérum physiologique (PBS, pH 7,4) ; on peut également utiliser différents agents pour augmenter leur viscosité.

Les anticorps conformes à l'Invention sont utilisés à des concentrations comprises entre 0,5 et 5 mg/ml, de préférence 1 mg/ml lorsqu'ils sont utilisés dans un but thérapeutique. De manière générale, les préparations thérapeutiques contenant les anticorps conformes à l'Invention sont administrés de façon systémique, quoique l'administration locale ne soit pas exclue.

Les anticorps monoclonaux conformes à l'Invention peuvent être non seulement utilisés en thérapeutique, mais aussi dans un but prophylactique.

Les anticorps conformes à l'Invention peuvent être utilisés isolément ; préférentiellement, ils seront utilisés ensemble. En effet, les Inventeurs ont constaté une synergie d'action entre les anticorps B-S12 et B-P8.

Les anticorps monoclonaux conformes à l'Invention peuvent également être utilisés comme réactifs de diagnostic pour identifier l'antigène gp130, ou un épitope de celui-ci, sur la surface des cellules ou dans des liquides biologiques. Pour de telles utilisations, les anticorps monoclonaux peuvent être couplés à des marqueurs, fluorescents, biotinylés, radioactifs ou autres. Il est également possible d'utiliser les anticorps monoclonaux conformes à l'Invention dans des tests ELISA ou RIA afin de doser gp130 dans des liquides biologiques. Les anticorps monoclonaux conformes à l'invention peuvent également être utilisés pour identifier et purifier gp130, ou des épitopes dudit récepteur, à partir de préparations de macromolécules susceptibles de contenir ledit récepteur ou ses épitopes, tels que par exemple, des lysats et fractions cellulaires, des préparations peptidiques ou oligosaccharidiques résultant par exemple de la digestion enzymatique d'une fraction cellulaire, ou bien obtenues par synthèse chimique.

Les anticorps monoclonaux conformes à l'Invention permettent également la production d'anticorps chimériques dont le domaine constant est d'origine humaine (immunoglobuline humaine), et la partie variable ou de préférence la partie hypervariable est d'origine murine (immunoglobuline murine). Pour le traitement des maladies dans lequel intervient gp130, ces anticorps chimériques peuvent être utilisés ou bien purs, ou bien couplés à des toxines, des substances radioactives, d'autres substances médicamenteuses, ou bien encapsulés dans des liposomes.

La présente Invention sera mieux comprise à l'aide du complément de description qui va suivre, qui se réfère à des exemples de préparation et d'utilisation des anticorps monoclonaux conformes à l'Invention.

Il va de soi, toutefois que ces exemples ne sont donnés qu'à titre d'illustration de l'objet de l'Invention, dont ils ne sauraient constituer en aucune manière une limitation.

### I. PREPARATION DES ANTICORPS MONOCLONAUX

### Exemple 1 - Immunisation, fusion, clonage et récolte des anticorps monoclonaux

Un protocole d'immunisation dérivé de celui décrit par MATTHEW et SANDROCK [J. Immunol. Methods, 100, 73-82, (1987)] a été utilisé.

Des souris femelles Balb/c ont subi une injection intrapéritonéale de 2 µg de gp130 soluble. Ce protocole a été répété trois fois, à intervalle de deux semaines, après quoi la quatrième injection a été faite par voie intraveineuse, et les splénocytes ont été extraits quatre jours plus tard et fusionnés. La fusion a été effectuée de la façon suivante : les splénocytes ont été fusionnés avec des cellules murines de myélome dénommées X63Ag8653 (le rapport splénocytes/cellules de myélome est de 5:1), en présence de polyéthylène glycol [KEARNEY et al., J. of Immunol., 123, 1548, (1978)]. La lignée cellulaire X63Ag8653 est déposée à la COLLECTION EUROPEENNE DE CULTURES DE CELLULES ANIMALES (ECACC), PHLS Centre of Applied Microbiology and Research, Porton Down, Salisbury, Wilshire, SP4 OJG, UK, sous le numéro de dépôt ECACC 850 114 20.

La suspension de cellules fusionnées a été lavée une fois, et cultivée sur milieu sélectif (RPMI 1640, 10% de sérum de cheval inactivé par la chaleur, 4 mM de glutamine, 13,6 mg/l d'hypoxanthine, 0,17 mg/l d'aminoptérine, et 10 µg/ml d'insuline).

Dix jours après la fusion, les surnageants des cultures dans lesquelles une croissance d'hybridomes a été observée ont été testés pour détecter la production d'anticorps monoclonaux anti-IL-6R.

Dans ce but, 75 µl de surnageant de chaque culture d'hybridomes ont été testés par cytométrie de flux sur les lignées cellulaires BAF (gp130 négative) et BAF130/80(gp130 positive).

Les hybridomes produisant des anticorps reconnaissant la lignée BAF130/80 ont été retenus et ont été clonés quatre fois, en utilisant la méthode de la dilution limite (densité d'ensemencement 0,2 cellules par puits de culture).

### Exemple 2 - Production in vivo et purification des anticorps monoclonaux

Les anticorps monoclonaux sont produits en grande quantité *in vivo* par injection intrapéritonéale des cellules d'hybridome B-S12 et B-P8 dans des souris Balb/c. Une semaine avant l'injection de cellules d'hybridome, les souris reçoivent une injection intrapéritonéale de 0,5 ml d'adjuvant de Freund incomplet. Le liquide d'ascite est récupéré 8 à 14 Jours après l'injection des cellules.

Les anticorps monoclonaux sont ensuite précipités à partir du liquide d'ascite par addition de sulfate d'ammonium (45 %), tamponnés à pH 7,7 par du Tris 0,02 mM et fixés à une colonne de Sépharose Q. Les anticorps fixés à la colonne sont lavés avec 1 % de Tween 20 dans 0,02 mM de Tris, pH 7,7 et ensuite élués de la colonne par une solution 0,02 mM Tris, 0,35 M NaCl, pH 7,7.

### II. - ACTIVITE BIOLOGIQUE DES ANTICORPS B-S12 ET B-P8

### Exemple 3 - Induction de la prolifération de la lignée cellulaire humaine IL-6 dépendante XG1, par les anticorps B-S12 et B-P8.

La lignée cellulaire humaine XG1 est une lignée de myélome, dont la prolifération et les propriétés dépendent de IL-6, et qui a été décrite par KLEIN [BLOOD, 74, 749, (1989)].

Pour les expérimentations, la lignée XG1 est cultivée pendant trois jours dans du milieu RPMI1640 en présence de 10 % de sérum de veau foetal et de β-mercaptoéthanol, ainsi que de :
- ou bien 100 pg (= 10 unités) d'IL-6 par puits de culture.;
- ou bien différentes concentrations des anticorps B-S12 ou B-P8.

Un témoin est constitué de cellules cultivées en l'absence d'IL-6 et d'anticorps.

Les cellules sont ensuite cultivées pendant 16 heures en présence de thymidine H³ qui s'incorpore dans l'ADN néosynthétisé, ce qui permet l'évaluation de la croissance cellulaire, puis elles sont collectées, et la radioactivité est mesurée dans un compteur béta.

Les mesures sont faites en parallèle, sur les cellules cultivées en l'absence d'IL-6 et en présence de différentes concentrations des anticorps B-S12 et/ou B-P8, sur les cellules cultivées sans anticorps en présence d'IL-6, et sur les cellules témoin.

La radioactivité moyenne mesurée sur les cellules témoin est de 8000 cpm.

La radioactivité moyenne mesurée dans des cellules cultivées en présence d'IL-6 sans anticorps est de 55000 cpm.

Les résultats obtenus en l'absence d'IL-6 et en présence de concentrations croissantes des anticorps B-S12 ou B-P8 sont indiqués dans le Tableau I ci-dessous :

**TABLEAU I**

| CONCENTRATIONS (ng) | B-S12 | B-P8 | B-P8 + 16ng B-S12 |
|---|---|---|---|
| | RADIOACTIVITE (CPM) | | |
| 2000 | 43720 | 40328 | 57342 |
| 400 | 44201 | 13031 | 57867 |
| 80 | 38782 | 8095 | 56816 |
| 16 | 32494 | 8203 | 52541 |
| 3,2 | 11383 | 8529 | 54785 |
| 0,64 | 9000 | 7690 | 45868 |
| 0,128 | 9600 | 7000 | 38921 |

Ces résultats montrent clairement que la croissance cellulaire est similaire dans le cas des cellules cultivées en présence des anticorps monoclonaux B-S12 et B-P8 et dans les cellules témoins cultivées en présence d'IL-6 sans anticorps. Ces deux anticorps stimulent donc, indépendamment de la présence d'IL-6, la croissance de la lignée cellulaire XG1. En outre,on observe clairement une synergie d'action entre ces 2 anticorps.

### Exemple 4 - Induction de la prolifération de la lignée cellulaire humaine TF-1 par l'anticorps B-S12.

La lignée cellulaire humaine TF-1 est une lignée érythroleucémique, qui a été décrite par KITAMURA et al. [J. Cell. Physiol. 140, 323-334 (1989)].

Pour les expérimentations, la lignée TF-1 est cultivée pendant trois jours dans du milieu RPMI1640 en présence de 10 % de sérum de veau foetal, ainsi que de :
- ou bien 2000 pg (= 10 unités) d'IL-6 par puits de culture.;
- ou bien différentes concentrations des anticorps B-S12 ou B-P8.

Un témoin est constitué de cellules cultivées en l'absence d'IL-6 et d'anticorps.

Les cellules sont ensuite cultivées pendant 16 heures en présence de thymidine H³ puis elles sont collectées, et la radioactivité est mesurée dans un compteur béta.

Les mesures sont faites en parallèle :
- sur les cellules cultivées en l'absence d'IL-6 et en présence de différentes concentrations des anticorps B-S12 ou B-P8,
- sur les cellules cultivées sans anticorps en présence d'IL-6,
- sur les cellules témoin.

La radioactivité moyenne mesurée sur les cellules témoin est de 1500 cpm.

La radioactivité moyenne mesurée dans des cellules cultivées en présence d'IL-6 sans anticorps est de 18000 cpm.

Les résultats obtenus en présence de concentrations croissantes des anticorps sont indiqués dans le Tableau II ci-dessous :

**TABLEAU II**

| CONCENTRATIONS (µg) | B-S12 | B-P8 |
|---|---|---|
| | RADIOACTIVITE (CPM) | |
| 10 | 11265 | 13260 |
| 1 | 10855 | 3077 |
| 0,1 | 6775 | 1800 |

Ces résultats montrent que des cellules cultivées en présence des anticorps monoclonaux B-S12, et B-P8 présentent une croissance cellulaire d'un ordre de grandeur comparable à celle des cellules témoins cultivées en présence d'IL-6 sans anticorps.

### Exemple 5 : Effet d'anticorps bloquant la prolifération induite par IL-6, sur la prolifération induite par B-S12 ET B-P8

La lignée cellulaire humaine XG1 est cultivée comme décrit à l'exemple 3 ci-dessus, en présence de :
- ou bien 40 pg (= 4 unités) d'IL-6 par puits de culture.;
- ou bien différentes concentrations des anticorps B-S12 et B-P8, en présence ou en l'absence de 10 µg de l'un ou l'autre des anticorps bloquants B-E8 (Anti-IL-6, décrit dans la Demande de Brevet Européen déposée sous le numéro 90 122694.4) ; ou B-R6 (Anti-IL-6R) : décrit dans la Demande de Brevet Francais publiée sous le numéro 2 694 767).

Un témoin est constitué de cellules cultivées en l'absence d'IL-6 et d'anticorps.

Les cellules sont ensuite cultivées et la radioactivité est mesurée comme décrit à l'exemple 3 ci-dessus.

La radioactivité moyenne mesurée sur les cellules-témoin est de 9000 cpm.

La radioactivité moyenne mesurée dans des cellules cultivées en présence de 4 unités d'IL-6 sans anticorps est de 60000 cpm ; la radioactivité moyenne mesurée dans des cellules cultivées en présence de 4 unités d'IL-6 et de 10 µg de l'anticorps B-E8 ou B-R6 est de 10000 cpm.

Les résultats obtenus pour différentes concentrations de B-S12 et B-P8 sont indiqués respectivement dans les Tableaux III et IV ci-dessous :

**TABLEAU III**

| CONCENTRATIONS | B-S12 | B-S12+B-E8 | B-S12+B-R6 |
|---|---|---|---|
| | RADIOACTIVITE (CPM) | | |
| 0 | - | 9340 | 9328 |
| 10µg | 67527 | 63589 | 63240 |
| 1µg | 58930 | 58773 | 58879 |
| 100ng | 49441 | 53158 | 50911 |
| 10ng | 40888 | 32280 | 31492 |
| 1ng | 14518 | 16654 | 20575 |
| 100pg | 10057 | 10516 | 12530 |

**TABLEAU IV**

| CONCENTRATIONS | B-P8 | B-P8+B-E8 | B-P8 B-R6 |
|---|---|---|---|
| | RADIOACTIVITE (CPM) | | |
| 0 | - | 10213 | 10200 |
| 10µg | 51363 | 49115 | 56268 |
| 1µg | 16405 | 13107 | 15702 |
| 100ng | 9526 | 10567 | 12094 |
| 10ng | 10309 | 9298 | 12322 |

Ces résultats montrent que la prolifération de la lignée cellulaire XG1 induite par B-S12 et B-P8 n'est pas bloquée par les anticorps B-E8 et B-R6, contrairement à la prolifération induite par l'IL-6

### Exemple 6 - Induction de la sécrétion d'haptoglobine par la lignée cellulaire humaine HepG2, par l'anticorps B-S12

Pour les expérimentations, la lignée d'hépatome HepG2 (ECACC n° 8501/1430 ; Désignation ATCC : HB8065), est cultivée en milieu ISCOVE pendant deux jours en présence de 10 % de sérum de veau foetal, et de dexaméthasone (10⁻⁶ M) ainsi que de :
- ou bien différentes concentrations d'oncostatine M (OM)par puits de culture.;
- ou bien différentes concentrations de l'anticorps B-S12 ou d'un anticorps monoclonal "non-pertinent" (c'est à dire reconnaissant un antigène sans relation avec gp130) de contrôle.

Un témoin est constitué de cellules cultivées en l'absence d'OM et d'anticorps.

Au bout de 48 heures de culture, les surnageants sont récoltés, et la concentration en est déterminée par ELISA.

Les résultats obtenus en présence de concentrations croissantes d'OM (exprimée en ng/ml) et des anticorps (exprimée en µg/ml) sont indiqués (en ng d'haptoglobine par ml) dans le Tableau V ci-dessous :

**TABLEAU V**

| Concentration en OM (ng/ml) ou en anticorps (µg/ml) | OM | B-S12 | AcM NON-PERTINENT |
|---|---|---|---|
| | Sécrétion d'haptoglobine(ng/ml) | | |
| 0 | 7,5 | 4 | 2 |
| 10⁻⁵ | 9 | 5 | 2.5 |
| 10⁻⁴ | 10 | 7 | 2 |
| 10⁻³ | 12,5 | 9 | 3 |
| 10⁻² | 15 | 12 | 4 |
| 10⁻¹ | 27 | 20 | 3 |
| 1 | 50 | 27 | 3,5 |
| 10 | - | 40 | 4 |

Ces résultats montrent une induction significative de la sécrétion d'haptoglobine dans les cellules cultivées en présence de l'anticorps monoclonal B-S12.

### Exemple 7 : Propriétés des fragments Fab de B-S12

Les fragments Fab de B-S12 sont obtenus par clivage à la papaïne.

### 1) RECONNAISSANCE DE GP130 PAR LES FRAGMENTS Fab

Des cellules BAF130/80 (gp130 positives) sont incubées pendant 30 minutes en présence de concentrations variables de Fab de B-S12, ou de B-S12. Après incubation, les cellules sont lavés 2 fois, et incubées avec du sérum de chèvre anti-souris marqué à la fluorescéine pendant 30 minutes, lavées à nouveau et analysées par cytométrie de flux.

Le Tableau VI ci-dessous représente le pourcentage de cellules marquées par chacun des différents réactifs (Fab ou anticorps complet), utilisés à des concentrations décroissantes.

**TABLEAU VI**

| CONCENTRATION (µg/ml) | B-S12 | Fab de B-S12 |
|---|---|---|
| 10 | 36 | 42 |
| 5 | 69 | 48 |
| 2,5 | 70 | 52 |
| 1,25 | 73 | 55 |
| 0,625 | 73 | 53 |
| 0,312 | 71 | 50 |
| 0,156 | 67 | 42 |
| 0,08 | 60 | 30 |
| 0,04 | 49 | - |
| 0,02 | 36 | 9 |
| 0,01 | 23 | - |
| 0,005 | 18 | - |

### 2) ACTIVITE BIOLOGIQUE DES FRAGMENTS Fab

L'activité biologique est déterminée sur des cellules XG1, selon le protocole décrit à l'Exemple 3.

Les mesures sont faites en parallèle, sur les cellules cultivées en l'absence d'IL-6 et en présence de différentes concentrations des fragments Fab et de l'anticorps complet B-S12, sur les cellules cultivées sans anticorps en présence d'IL-6, et sur les cellules témoin.

La radioactivité moyenne mesurée sur les cellules témoin est de 4000 cpm.

La radioactivité moyenne mesurée dans des cellules cultivées en présence d'IL-6 sans anticorps est de 44000 cpm.

Les résultats obtenus en présence de concentrations décroissantes des fragments Fab et de l'anticorps complet B-S12 sont indiqués dans le Tableau VII ci-dessous :

**TABLEAU VII**

| CONCENTRATIONS (ng) | B-S12 | Fab de B-S12 |
|---|---|---|
| | RADIOACTIVITE (CPM) | |
| 10000 | 30466 | 2826 |
| 2000 | 29157 | 3100 |
| 400 | 31008 | 4200 |
| 80 | 27372 | 4990 |
| 16 | 22038 | 4040 |
| 3,2 | 9240 | 4360 |
| 0,64 | 4296 | 4200 |

Ces résultats montrent que les fragments Fab, bien que reconnaissant l'antigène gp130, sont incapables d'induire la croissance de la lignée cellulaire XG1.

### Exemple 8 : Analyse des phosphorylations induites par l'anticorps B-S12

La lignée de neuroblastome humaine SK-N-MC [BIEDLER J.L., HELRON et SPENGLER, B.A. CANCER RESEARCH, 33, p. 2643, (1973)] exprime fortement la sous-unité de transduction gp130 des récepteurs IL-6, IL-11, LIF, CNTF, OM et cardiotrophine I.

Lors de la fixation des ligands sur leurs récepteurs, la chaîne gp130 est très rapidement phosphorylée par des kinases sous-membranaires de la famille JAK [STAHL et al., Science, 263, p. 92, (1994)] pour pouvoir accepter la fixation de protéines de la famille STAT [IHLE et KERR, TIG, 11:2 69-74, (1995)]. Ces dernières, après contact avec gp130, sont ensuite transloquées vers le noyau pour y délivrer l'information afférente [STAHL et al., Science, 267, p. 1349, (1995)].

Les cellules SK-N-MC en phase exponentielle de culture sont privées de sérum pendant 5 h pour ralentir les phénomènes endogènes de phosphorylation constitutive.

Une cytokine (OM, 50 ng/ml) ou l'anticorps B-S12 sont ajoutés aux cultures respectivement pendant :
- 10 minutes et 20 minutes pour une première expérimentation, ou
- 5 à 30 minutes pour une seconde expérimentation.

Le milieu de culture (RPMI 1640) est ensuite enlevé et remplacé par 1 ml de tampon de lyse (Tris 10 mM, pH 7.6, 5 mM EDTA, 30 mM de pyrophosphate de sodium, 1 mM de fluorure de sodium, 1 mM orthovanadate, 1% TRITON X100). Les échantillons sont ensuite mis sous agitation pendant 1 h, avant d'être centrifugés (30 minutes, 12000 rpm, 4°C).

Le culot est éliminé, et gp130, ou les kinases JAKs, sont immunoprécipitées à l'aide respectivement d'anticorps anti-gp130 (10 µg/puits) ou d'anticorps polyclonaux anti-JAK1 et anti-JAK2 (UBI ; distribués par EUROMEDEX, BP80, 67460 SOUFFELWEYERSHEIM, FRANCE) dilués au 1/100. Le complexe Ac-Ag est isolé en ajoutant 20 µl d'une solution de billes recouvertes de protéine A. Après 2 h de contact les billes sont lavées 4 fois par centrifugation avec le tampon de lyse, avant d'être reprises dans le tampon d'échantillon de LAEMLI, contenant du β-ME comme agent réducteur. L'échantillon est chauffé 10 minutes à ébullition pour relibérer les constituants du complexe immunoprécipité, avant d'être chargé sur un gel conventiel d'électrophorèse SDS-PAGE à 7.5%. Après une migration de 2h les échantillons sont transférés sur une membrane d'IMMOBILON 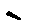 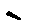 (AMERSHAM) par électrotransfert.

Après saturation des sites d'adsorption non-spécifique par incubation pendant une nuit en présence d'une solution 200 mM NaCl, 50 mM Tris, pH 7.6, 6% BSA, la membrane est incubée pendant 4h en présence d'un anticorps reconnaissant le motif phosphotyrosine 4G10 (UBI) à 0.5 µg/ml. Après 4 lavages en tampon 200 mM NaCl, 50 mM Tris, pH 7.6, 0.05% Tween 20, un anticorps anti-immunoglobuline de lapin couplé à la peroxydase (TAGO ; distribué par BIOSOFT, 60 rue de Wattignies, 75580 PARIS CEDEX 12, FRANCE) est ajouté à une dilution de 1/2000.

Après 1h de contact, la membrane est lavée 5 fois, puis révélée avec le kit ECL de chez AMERSHAM (RPN 2106). L'émission de photons générée est visualisée à l'aide d'un film AMERSHAM RPN 2103.

Un signal de phosphorylation est détectable à partir d'une concentration en anticorps de 5 µg/ml ; ce signal augmente en intensité jusqu'à 50 µg/ml (concentration la plus forte testée).

Une analyse de la cinétique du phénomène montre que celui-ci atteint une intensité maximale après 20 minutes de contact avant de diminuer après 30 minutes. Un anticorps contrôle d'isotype IgG1 incubé avec les cellules pendant 20 minutes est sans aucun effet sur l'activation de gp130. Le phénomène observé est donc bien dépendant de la partie variable de l'anticorps B-S12.

Ces expériences montrent que l'anticorps B-S12 est capable d'induire, de manière dose-dépendante, une phosphorylation de la protéine de transduction du signal gp130.

D'autres résultats montrent que l'anticorps B-S12 est effectivement capable de stimuler l'autophosphorylation des 2 kinases sous-membranaires, JAK1 et 2, impliquées dans les phénomènes de phosphorylation de gp130, de manière similaire à ce qui a été rapporté pour les ligands [STAHL et al., Science, 263, p. 92, (1994)].

### Exemple 9 : Mesure de GP130 soluble par Elisa Sandwich

Le protocole opératoire est le suivant :
- Incubation des plaques de microtitration avec les anticorps B-S12 ou B-P4 (AcM anti-gp130 reconnaissant un autre épitope que B-S12) (0,5 µg/puits dans 100 µl de tampon PBS) pendant une nuit à 4°C.
- Saturation avec du tampon PBS à 5% d'albumine pendant 90 minutes à température de la pièce.
- Distribution dans chaque puits de la plaque de 100 µl de sérum humain à des dilutions variables (le sérum humain contient du gp130 soluble) et de 50 µl d'anticorps B-S12 ou B-P4 biotinylé, et incubation pendant 2h à température ambiante.
- Distribution dans les puits d'une solution de streptavidine marquée à la peroxydase, puis de son substrat TMB (TMB = 3,3',5,5'-tétraméthylbenzidine) et mesure de la densité optique à 450 nm après blocage de la réaction par 100 µl d'H₂SO₄ 1N.

Les résultats exprimés en densité optique (DO) sont reportés dans le Tableau VIII ci-dessous, pour chacune des combinaisons d'anticorps.

**TABLEAU VIII**

| SERUM (dilution) | BP4 + BP4_{biot} | BS12 + BS12_{biot} | BP4 + BS12_{biot} | BS12 + BP4_{biot} |
|---|---|---|---|---|
| Pur | 0,213 | 0,452 | >2,800 | >2,800 |
| 1/10 | 0,277 | 0,123 | 2,220 | 1,956 |
| 1/100 | 0,240 | 0,135 | 2,080 | 1,870 |
| 0 | 0,236 | 0,143 | 0,134 | 0,138 |

BS-12 permet de mesurer le gp130 soluble du sérum humain pur et dilué 1/10 et 1/100 à condition d'être utilisé en combinaison avec un anticorps reconnaissant un autre épitope de gp130.

Ces résultats montrent que l'épitope reconnu par B-S12 n'est pas répété sur le gp130 soluble. Le signal peu élevé observé dans le cas de l'utilisation de la combinaison BS12 + BS12_{biot} avec du sérum humain pur reflète la faible proportion de formes dimères de gp130.

## Revendications

1. Anticorps monoclonal dirigé contre le récepteur gp130, caractérisé en ce qu'il est choisi dans le groupe constitué par:
- l'anticorps monoclonal d'isotype IgG1 dénommé B-S12, produit par la lignée d'hybridome déposée le 12 avril 1995 auprès de la C.N.C.M. sous le numéro de dépôt I-1561 , et
- l'anticorps monoclonal d'isotype IgG1 dénommé B-P8, produit par la lignée d'hybridome déposée le 12 avril 1995 auprès de la C.N.C.M., sous le numéro de dépôt I-1560.

2. Utilisation d'un anticorps selon la revendication 1, pour l'obtention de médicaments.

3. Utilisation selon la revendication 2, caractérisée en ce que lesdits médicaments sont destinés au traitement ou à la prophylaxie de maladies impliquant la gp130.

4. Utilisation d'un anticorps selon la revendication 1, pour induire la dimérisation de gp130.

5. Utilisation d'un anticorps selon la revendication 1, comme adjuvant pour la culture de cellules possédant le récepteur gp130.

6. Utilisation d'un anticorps selon la revendication 1, comme réactif de diagnostic.

7. Compositions pharmaceutiques, caractérisées en ce qu'elles comprennent, en tant que principe actif au moins un anticorps selon la revendication 1.
